# EUROPEAN PATENT SPECIFICATION

(11) **EP 2 915 557 B1**
(45) Date of publication and mention of the grant of the patent: **09.05.2018**
(21) Application number: 13850016.0
(22) Date of filing: 01.11.2013
(51) Int. Cl.: A61M 37/00, A61K 9/10, A61K 31/05, A61K 31/203, A61K 47/36, A61P 17/00

(54) **RETINOIC ACID MICRONEEDLE**
RETINSÄURE-MIKRONADEL
MICRO-AIGUILLE D'ACIDE RÉTINOÏQUE

(30) Priority: 02.11.2012 JP 2012253664
(43) Date of publication of application: 09.09.2015
(73) Proprietor: Cosmed Pharmaceutical Co., Ltd., Kyoto-shi, Kyoto 601-8014 (JP); Osaka University, Suita-shi, Osaka 565-0871 (JP); Public University Corporation Nara Medical University, Nara 634-8521 (JP)
(72) Inventor: QUAN, Ying-shu, Kyoto-shi Kyoto 601-8014 (JP); KAMIYAMA, Fumio, Kyoto-shi Kyoto 601-8014 (JP); SAITO, Mio, Kyoto-shi Kyoto 601-8014 (JP); NAKAGAWA, Shinsaku, Suita-shi Osaka 565-0871 (JP); OKADA, Naoki, Suita-shi Osaka 565-0871 (JP); HIROBE, Sachiko, Suita-shi Osaka 565-0871 (JP); ASADA, Hideo, Kashihara-shi Nara 634-8521 (JP)
(74) Representative: Ter Meer Steinmeister & Partner
(86) International application number: PCT/JP2013/079665
(87) International publication number: WO 2014/069615

(56) References cited:
- WO-A1-2013/122160
- JP-A- 2007 130 417
- JP-A- 2008 284 318
- JP-A- 2010 082 401
- JP-A- 2010 209 023
- JP-B2- 2 794 252
- JP-B2- 4 521 492
- US-A1- 2008 319 404
- YASUHIRO HIRAISHI ET AL: "Performance and characteristics evaluation of a sodium hyaluronate-based microneedle patch for a transcutaneous drug delivery system", INTERNATIONAL JOURNAL OF PHARMACEUTICS, vol. 441, no. 1-2, 5 November 2012 (2012-11-05), pages 570-579, XP055191792, ISSN: 0378-5173, DOI: 10.1016/j.ijpharm.2012.10.042

## Description

### Technical Field

The present invention relates to a microneedle retaining retinoic acid.

### Background Art

Although studies for improving effectiveness and decreasing side effects of external medicines for treating pimples and stains have progressed, a sufficiently effective medicine is yet unknown. A treatment method of freezing a lesion by liquid nitrogen has risks of causing intense inflammation after the treatment and remaining scar, while it is covered by insurance. A treatment method of evaporating and scraping a lesion from a skin surface by using a carbon dioxide laser allows cure without trace, but it is not covered by insurance and results in a great burden of medical costs on a patient.

Retinoic acid is also called vitamin A and is a retinoid having a carboxylic acid group at its terminal. Retinoic acid acts to remove corneum of the skin, to enhance division and proliferation of epidermal cells, and to promote skin regeneration. Thus, excretion of melamine around a basal epidermal layer is enhanced. As a result, retinoic acid is effective in treating pimples and stains (senile pigment freckle, seborrheic keratosis, etc.) and also in improving wrinkles. These effects are considerably increased by combining with a bleaching agent such as hydroquinone.

Retinoic acid is very instable and particularly has drawbacks of being sensitive to light and heat and thus easy to be decomposed. Furthermore, since retinoid has intensive dermal irritancy, it is difficult to transdermally administer retinoid alone. Thereby, these properties have prevented these compounds from being widespread as external preparations. It has been proposed that retinoid is enclosed in a microcapsule when transdermally administered in order to reduce decomposition and skin irritation of retinoid (Patent Document 1).

It is not easy to effectively use an external medicine containing retinoic acid for pimples and stains, and researches aimed at improving effectiveness and reducing side effects have progressed, but a sufficiently effective medicine is yet unknown.

Skin is consistently exposed to crises of invasion of pathogens from the outside world, and forms a front-line defense mechanism by comprising a physical barrier as a corneum and an immune barrier mainly consisting of Langerhans cells residing in its underlying epidermal layer. Consequently, it is not easy to supply drugs and cosmetics to a living epidermal layer by breaking through this defense mechanism. Therefore, the efficiency of transdermal administration of drugs is low when just applied on the surface of the skin.

As a method which solves these problems and surely supplies medicinal ingredients to a specific site beneath the skin, a microneedle has been proposed (Patent Document 2). Since the microneedle is very fine, pain and bleeding are not caused in its insertion into the stratum corneum, and puncture wound is rapidly closed, therefore the method is preferable as a method for surely supplying a drug beneath the skin. Note that a means having a plurality of microneedles on a substrate is called a microneedle array. Furthermore, a microneedle array having an adhesive tape or the like for fixing the microneedle array on the skin is called a microneedle patch.

As a material for the microneedle, a substance which dissolves and disappears in a living body has been proposed (Patent Document 3). When making such a microneedle contain a vaccine and inserting it into a skin, the microneedle dissolves and disappears in the skin, and thus the vaccine can be surely supplied to a specific site of the skin. As a substance which dissolves and disappears in a living body, polysaccharides such as hyaluronic acid, collagen and gelatin have been proposed (Patent Document 4).

### Prior Art Documents

### Patent Documents

[Patent Document 1] JP-T-2007-536259
[Patent Document 2] JP-T-2002-517300
[Patent Document 3] JP-A-2003-238347
[Patent Document 4] JP-A-2008-284318
JP-A-2010-82401 discloses a microneedle, wherein a drug is contained in a microneedle material.

### Summary of Invention

### Technical Problem

The problem to be solved by the present invention is to provide a microneedle array which can effectively supply retinoic acid to the skin and is effective for treating pimples, pigmentation or wrinkles.

### Solution to Problem

A retinoic acid microneedle array according to the present invention comprises: a microneedle array having a microneedle substrate and microneedles arranged on the microneedle substrate; and a drug retained on the microneedles and containing a water-swelling polymer and retinoic acid, wherein the microneedle array comprises the water-swelling polymer or a thermoplastic, a tip of the microneedle projects through a discontinuous face, and the drug is retained on the tip, wherein a concentration of the retinoic acid in the drug is 0.01 to 10 wt%, and wherein the retinoic acid is surrounded by the water-swelling polymer to turn into the drug. Herein, the water-swelling polymer means a polymer which swells by adding a small amount of water and dissolves in a large amount of water.

Specific constitutions for retaining the drug prepared by blending the water-swelling polymer and the retinoic acid on the microneedles may include the following three configurations.
(1) A configuration in which the microneedles comprise the drug containing the water-swelling polymer and the retinoic acid (a uniform type). The retinoic acid is uniformly retained on the microneedles.
(2) A configuration in which the microneedle array comprises the water-swelling polymer, a tip of the microneedle projects through a discontinuous face, and the drug is retained on the tip (a tip retaining-water swelling polymer type).
(3) A configuration in which the microneedle array comprises a thermoplastic, a tip of the microneedle projects through a discontinuous face, and the drug is retained on the tip (a tip retaining-thermoplastic polymer type).

In any of the above three configurations, the retinoic acid is surrounded by the water-swelling polymer to turn into a drug, and thereby the stability of the retinoic acid is considerably improved. The reason for the improved stability seems to be that the retinoic acid, which is easily decomposed by heat, light and oxygen, is blocked from oxygen by being surrounded by the water-swelling polymer.

Furthermore, an uptake speed of the retinoic acid into the body can be regulated by regulating the dissolution rate of the water-swelling polymer surrounding the retinoic acid, and thereby side effects can be reduced.

A concentration of the retinoic acid in the drug is 0.01 to 10 wt%. If the amount is less than this concentration, the efficacy may be hardly expressed, and if the amount is more than this concentration, a compatibility with the water-swelling polymer is poor, and the retinoic acid may precipitate on a surface.

When the drug contains 0.01 to 30 wt% of hydroquinone, the effects of the retinoic acid are further enhanced. That is because, when the content of hydroquinone is less than this concentration, the effects may be hardly obtained, and when the content is more than this concentration, skin irritation may be increased by side effects of hydroquinone. Also, addition of vitamin C and its derivative is preferable because they are effective for further enhancing the effects of the retinoic acid. A concentration of vitamin C and its derivative is preferably 0.10 to 30 wt% in the drug.

In the present invention, the water swelling polymer is not particularly limited, and hyaluronic acid, chondroitin sulfate, hydroxypropyl cellulose, dextran, proteoglycan or the like can be preferably used. More preferably, a main ingredient is hyaluronic acid.

A length of the microneedle is preferably 150 to 1000 µm. A typical area of the substrate of the microneedle array is preferably 0.5 to 40 cm².

The shape of the microneedle array may be various shapes such as circle, oval, square, rectangle, crescent shape, comma shape, triangle, star shape, face mask shape according to the application site. Herein, the comma shape may include a conventional comma shape as well as a symmetric shape.

### Advantageous Effects of Invention

Since retinoic acid can be injected through the corneum by retaining the retinoic acid on the microneedle, the epidermal permeation efficiency is high and the retinoic acid can directly reach and act on the skin. Therefore the effects can be exhibited even by a far smaller amount of retinoic acid than in the case of just application on the skin. Side effects can be controlled by adjusting the uptake speed with the microneedles.

By making the retinoic acid which is easy to decompose by heat, light and oxygen surrounded by the water-swelling polymer, the retinoic acid can be blocked from oxygen to enhance the stability. The decomposition of retinoic acid observed in application on the skin can be avoided, and thus great effects can be obtained.

### Brief Description of Drawings

Fig. 1 shows a schematic sectional view of a part of the microneedle array in Example 1.
Fig. 2 shows a schematic sectional view of a part of the microneedle array in Example 2.

### Description of Embodiments

Although examples of the present invention will be explained in detail below, but the present invention is not limited to these examples. Note that, in the following examples, the hyaluronic acid (molecular weight: 800,000, trade name: FCH-80LE) was purchased from Kikkoman Biochemifa Company, the dextran (trade name: Dextran 70) was purchased from Nippon Bulk Yakuhin Co., Ltd., and the retinoic acid and the hydroxypropyl cellulose were purchased from Wako Pure Chemical Industries, Ltd.

### Example 1

### (Process for making a tip retaining-thermoplastic polymer type microneedle array)

Nylon 12 was used as a material and injected into a mold to produce a microneedle array. In order to retain a drug on the tip of the microneedle, a discontinuous face is provided on the tip. To provide the discontinuous face on the tip of the microneedle, a discontinuous face should be previously provided on the mold. The mold can be produced by electrocasting after a microneedle pattern is formed by using a lithography method in which a photosensitive resin is light-irradiated. When an X-ray photosensitive resin is used, a microneedle pattern is formed by irradiation with synchrotron X-ray, and then electrocasted to produce a mold. The height of the microneedle was 0.6 mm, in which the length of the part above the discontinuous face (the tip part) was 0.2 mm. The size of the margin of the discontinuous face was 0.03 mm.

As a water-swelling polymer, 8% aqueous solution of hydroxypropyl cellulose:dextran (weight ratio 7:3) was used. The retinoic acid was dissolved in a small amount of ethanol, and mixed with the 8% aqueous solution of water-swelling polymer. The concentration of the retinoic acid in the mixture was 0.08 wt%. Thus, 1 wt% of retinoic acid is contained in the drug.

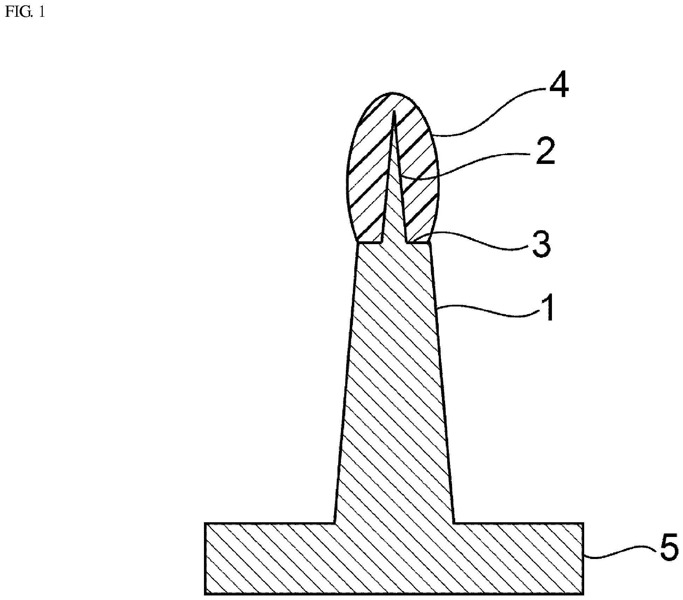

In order to retain the retinoic acid on the tips, the tips of the microneedles of the microneedle array were soaked in the mixture of the water-swelling polymer and the retinoic acid. The microneedles were drawn out and dried to produce a microneedle array having microneedles retaining the drug on the tip. A cross-section shape of this microneedle array is shown in Fig. 1. Note that Fig. 1 shows one microneedle part chosen from the microneedle array in Example 1. As shown in Fig. 1, the microneedle 1 is placed on the microneedle substrate 5. The drug 4 is retained on the tip 2 which projects through a discontinuous face 3 in a state as shown in Fig. 1.

This microneedle array was cut into a circular shape with a diameter of 1 cm, and lined with a sheet prepared by applying an adhesive agent on one side of a circular PET with a diameter of 2 cm (16 µm thickness) to produce a microneedle patch.

This microneedle array retains the drug 4 on the tip 2 of the microneedle made of a thermoplastic, and it was confirmed by a microscope that the drug 4 was retained on the side of the tip 2 from the discontinuous face 3.

### Example 2 (not according to the invention)

### (Process for making a uniform type microneedle array)

A mold for forming the microneedle was produced by a lithography method. After a prescribed shape of microneedle pattern was formed by light-irradiating a photosensitive resin, a concave portion for forming the microneedle to which the prescribed shape of microneedle pattern was transferred was formed by electrocasting to produce the mold.

The retinoic acid dissolved in a small amount of ethanol was added to an aqueous solution of hyaluronic acid with a solid content of 5%, and mixed. The concentration of the retinoic acid in the aqueous solution was 0.01%. The mold was filled with the mixed aqueous solution at room temperature, the solution was dried by evaporation of moisture and then removed to produce the microneedle array. The microneedle array was cut into a circular shape with a diameter of 1 cm. The concentration of the retinoic acid in the drug was 0.2 wt%.

Each microneedle is a circular truncated cone shape with a base diameter of 0.2 mm, a tip diameter of 0.04 mm, a height of 0.8 mm, and they are arranged in a reticular pattern at 0.6 mm interval. This microneedle array includes 250 microneedles per 1 cm². Hereinafter, the microneedle array of this shape will be referred to as 800-MN. This microneedle array was lined with a sheet prepared by applying an adhesive agent on one side of a circular PET with a diameter of 2 cm (16 µm thickness) to produce a microneedle patch.

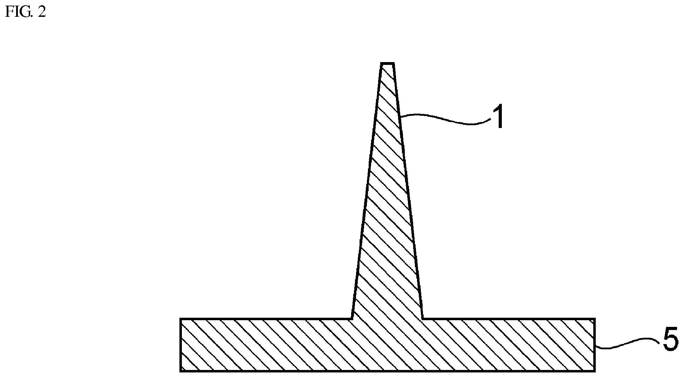

A schematic drawing of a cross-section shape of one microneedle constituting this microneedle array is shown in Fig. 2.

The whole microneedle array is constituted with a drug in which the water-swelling polymer and the retinoic acid are mixed. The content of the retinoic acid in the needle part was 5 µg.

The two retinoic acid microneedle arrays produced in Examples 1 and 2 are summarized and compared in the following Table 1. The content of the retinoic acid represents the content thereof in the microneedle array needle.

**Table 1**

| Examples | Microneedle array | Water-swelling polymer | Content of retinoic acid | Hight of microneedle |
|---|---|---|---|---|
| 1 | Tip retaining-water swelling polymer type | Hydroxypropyl cellulose : Dextran (7:3) | 2.0 µg | 0.6 mm |
| 2 | Uniform type | Hyaluronic acid | 5 µg | 0.8 mm |

### Example 3

The two retinoic acid microneedle arrays produced in Examples 1 and 2 were applied to the shaved skin of male Wistar rats. Two hours after the application, the microneedle arrays were removed, and the tips of the needles were observed by a microscope. Then, the needles in the uniform-type microneedle array thoroughly dissolved and disappeared. The drug contained in the needles on the thermoplastic polymer-containing tip type microneedle array thoroughly disappeared. Hence, it was understood that the whole retinoic acid contained in the needles of both microneedles was delivered into the skin.

### Example 4 (not according to the invention)

Retinoic acid microneedles containing hydroquinone were prepared by using two molds. The process was the same as in Example 2.

Microneedle 1: a circular truncated cone shape with a base diameter of 0.2 mm, a tip diameter of 0.04 mm and a height of 0.8 mm, arranged in a reticular pattern at 0.6 mm interval.

Microneedle 2: a konide shape with a tip diameter of 0.02 mm and a height of 0.3 mm, and arranged in a reticular pattern at 0.6 mm interval.

Both microneedle arrays were prepared so as to have diameters of 1 cm. The concentration of the retinoic acid in the drug was 0.4 wt%, and the concentration of the hydroquinone was 0.1 wt%.

A clinical trial was carried out in volunteers by using the microneedles of the present invention.

### (Result 1)

A subject was a 46-year-old woman who had a dark brown macule of about 1 cm² below the right eye, to which the microneedle 2 was applied.

In the first month, it was applied twice a week, and in the second month, once a week.

One week after starting the application, the color of the macule on the application site became dense.

Two weeks later, skin peeling and redness were observed at the application site.

At the third week, the color of the macule on the application site paled, and the area of the macule was decreased.

At the fourth and fifth weeks, the color of the macule on the application site paled.

### (Result 2)

A subject was a 51-year-old man who had a black blotch with a diameter of 2 mm on the corner of the left eye, to which the microneedle 2 was applied for 1 month.

A total of 7 administrations were carried out twice a week.

One week after starting the application, redness was observed on the application site of the skin.

At the second week, the color of the macule on the application site of the skin paled.

At the third week, slight skin peeling on the blotch was observed on palpation. Redness was observed on the skin.

At the fourth week, scab was observed, and the black color on the blotch faded.

### (Result 3)

A subject was a 46-year-old woman who had a brown wart above the corner of the right eye, to which the microneedle 1 was applied for two months. A total of 11 administrations were carried out twice a week, and from the second month, once a week.

At the second week, the application site of the skin showed no change. The subject felt pruritus on the application site.

At the third week, the wart decreased, and the brown color of the wart paled.

At the fourth and fifth weeks, the wart further decreased, the color of the wart paled.

### Reference Numerals

- 1: microneedle
- 2: tip
- 3: discontinuous face
- 4: drug
- 5: microneedle substrate

## Claims

1. A retinoic acid microneedle array comprising:
a microneedle array having a microneedle substrate and a plurality of microneedles arranged on the microneedle substrate; and
a drug retained on the microneedles and containing a water-swelling polymer and retinoic acid, wherein
the microneedle array comprises the water-swelling polymer or a thermoplastic,
a tip of the microneedle projects through a discontinuous face, and
the drug is retained on the tip, wherein
a concentration of the retinoic acid in the drug is 0.01 to 10 wt%, and
wherein the retinoic acid is surrounded by the water-swelling polymer to turn into the drug.

2. The retinoic acid microneedle array according to Claim 1, wherein
the drug contains at least one selected from a group consisting of hydroquinone, vitamin C and derivative of the vitamin C, at a concentration of 0.10 to 30 wt%.

3. The retinoic acid microneedle array according to Claim 1 or 2, wherein
a main ingredient of the water-swelling polymer is hyaluronic acid.

## Patentansprüche

1. Retinsäure-Mikronadelanordnung, umfassend:
eine Mikronadelanordnung mit einem Mikronadelsubstrat und einer Vielzahl von auf dem Mikronadelsubstrat angeordneten Mikronadeln; und
ein Arzneimittel, das auf den Mikronadeln zurückgehalten wird und ein wasserquellendes Polymer und Retinsäure enthält, wobei
die Mikronadelanordnung das wasserquellende Polymer oder einen Thermoplast umfasst,
eine Spitze der Mikronadel durch eine diskontinuierliche Fläche heraussteht, und
das Arzneimittel auf der Spitze zurückgehalten wird, wobei
eine Konzentration der Retinsäure in dem Arzneimittel 0,01 bis 10 Gew.-% beträgt, und
wobei die Retinsäure von dem wasserquellenden Polymer umgeben wird, um zu dem Arzneimittel zu werden.

2. Retinsäure-Mikronadelanordnung nach Anspruch 1, wobei das Arzneimittel mindestens eines enthält, gewählt aus einer Gruppe, bestehend aus Hydrochinon, Vitamin C und Vitamin C-Derivat, in einer Konzentration von 0,10 bis 30 Gew.-%.

3. Retinsäure-Mikronadelanordnung nach Anspruch 1 oder 2, wobei ein Hauptbestandteil des wasserquellenden Polymers Hyaluronsäure ist.

## Revendications

1. Réseau de micro-aiguilles d'acide rétinoïque comprenant:
une matrice de micro-aiguilles ayant un substrat de micro-aiguille et une pluralité de micro-aiguilles disposées sur le substrat de micro-aiguille; et
un médicament retenu sur les micro-aiguilles et contenant un polymère gonflant à l'eau et de l'acide rétinoïque, dans lequel
le réseau de micro-aiguilles comprend le polymère gonflant à l'eau ou un thermoplastique,
une pointe de la micro-aiguille faisant saillie à travers une face discontinue, et
le médicament est retenu sur la pointe, dans lequel
une concentration de l'acide rétinoïque dans le médicament est de 0,01 à 10% en poids, et
dans lequel l'acide rétinoïque est entouré par le polymère gonflant à l'eau pour devenir le médicament.

2. Le réseau de micro-aiguilles d'acide rétinoïque selon la revendication 1, dans lequel le médicament contient au moins un composé choisi parmi un groupe constitué par l'hydroquinone, la vitamine C et un dérivé de la vitamine C, à une concentration de 0,10 à 30% en poids.

3. Le réseau de micro-aiguilles d'acide rétinoïque la revendication 1 ou 2, dans lequel un ingrédient principal du polymère gonflant dans l'eau est l'acide hyaluronique.
